(19) Europäisches Patentamt
European Patent Office
Office européen des brevets

(11) EP 3 805 209 A1

(12) EUROPEAN PATENT APPLICATION
published in accordance with Art. 153(4) EPC

(43) Date of publication:
**14.04.2021 Bulletin 2021/15**

(21) Application number: **20760292.1**

(22) Date of filing: **18.02.2020**

(51) Int Cl.:
***C07D 317/38*** *(2006.01)* ***B01J 31/22*** *(2006.01)*

(86) International application number:
**PCT/CN2020/075766**

(87) International publication number:
**WO 2020/169035 (27.08.2020 Gazette 2020/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.02.2019 CN 201910122112**

(71) Applicant: **Shandong Shida Shenghua Chemical Group Co., Ltd**
**Shandong 257503 (CN)**

(72) Inventors:
- **GUO, Jianjun**
**Shandong 257503 (CN)**
- **LI, Xin**
**Shandong 257503 (CN)**
- **ZHANG, Xingming**
**Shandong 257503 (CN)**

(74) Representative: **Ter Meer Steinmeister & Partner Patentanwälte mbB**
**Nymphenburger Straße 4**
**80335 München (DE)**

# (54) METHOD FOR PREPARING ALKYLENE CARBONATE FROM ALKYLENE OXIDE AND CARBON DIOXIDE

(57) The present disclosure relates to a method for preparing alkylene carbonate from alkylene oxide and carbon dioxide. The method comprises: contacting a raw material comprising alkylene oxide and carbon dioxide with a catalyst by contact to generate alkylene carbonate; wherein the catalyst is at least one selected from the complexes formed from multidentate ligand and zinc; wherein the multidentate ligand comprises imidazole-based compound and salicylic acid-based compound; a molar ratio of the zinc, the imidazole-based compound and the salicylic acid-based compound in the catalyst is any ratio in a range of 5:1-50: 1-50. The beneficial effects achieved are as follows: the method adopts a complex catalyst system formed from multidentate ligand and zinc. The method achieved the advantages of high conversion efficiency of alkylene oxide, high selectivity of alkylene carbonate, and thus achieved good technical effect. The method for preparing the catalyst system is simple, with low cost, and the catalyst achieves good activity, and can be popularized to the industrial production of alkylene carbonate.

EP 3 805 209 A1

## Description

### TECHNICAL FIELD

The present disclosure relates to a method for preparing alkylene carbonate, in particular to a method for preparing the alkylene carbonate from alkylene oxide and carbon dioxide.

### BACKGROUND

The alkylene carbonate is an excellent solvent, a fine chemical intermediate, and a potential basic raw material for organic chemicals. Meanwhile, $CO_2$ is a greenhouse gas. How to effectively fix $CO_2$ becomes one of the most challenging problems in the century, while it is a good fixation method for synthesizing alkylene carbonate by a reaction between alkylene oxide and $CO_2$. With increasing interest in the co-production of dimethyl carbonate and diols starting from alkylene carbonates recently, the fixation of $CO_2$ by cyclic carbonates has also been paid increasing attention.

It has been reported that there are various catalyst systems for an esterification reaction of $CO_2$ and EO, comprising ammonium onium halides, phosphonium onium halides, tributyl methyl phosphonium iodide, metal halides (such as KBr, $ZnCl_2$, etc.), imidazole-based and pyridine-based ionic liquids, metal complexes, amines, and polymer supported catalysts, etc. There are some disadvantages in these catalyst systems, such as low reaction activity or yellowing of obtained product, thereby resulting in degraded quality of the product. US2907771 discloses the followings: Olin Mathieson, a US company, found that an EC solution of alkaline earth metal salt $CaCl_2$ has a better catalytic activity in a cycloaddition reaction of EO, and an EC yield of 68.4% is achieved in a continued reaction at 800 Psi and 190 °C. US7488835 disclosed the followings: Shell Group of Companies studied the effect of the types of alkali metal and complex on the catalysts for catalyzing EC synthesis reaction from EO, and found that the best effect was achieved when the catalyst system was KI/18-crown-6, and an EO conversion efficiency of 84% and a EC selectivity of 98% were achieved under the reaction condition of 2MPa, 80 °C for 1 h. CN1995032 discloses the followings: ionic liquid of 1-methyl-3-butylimidazole bromide was used as catalyst, and an EC yield of 92.2 % was achieved under the reaction condition of EO/Cat = 208, 100 °C and 2.0MPa for 0.7 h .

We herein prepared a catalyst system with multidentate complex compound, which comprises imidazole group, salicylic acid group and zinc, and achieved a catalyst with good activity.

### SUMMARY

In view of the above disadvantages of the prior art, the object of present disclosure is to provide a method for preparing alkylene carbonate from alkylene oxide and carbon dioxide, which is characterized by high catalyst activity.

The disclosure provides a method for preparing alkylene carbonate from alkylene oxide and carbon dioxide, comprising:

> contacting a raw material comprising alkylene oxide and carbon dioxide with a catalyst to produce alkylene carbonate;
> wherein the catalyst is at least one selected from complexes formed from multidentate ligand and zinc;
> wherein the multidentate ligand comprises imidazole-based compound and salicylic acid-based compound; and
> wherein, a molar ratio of zinc, the imidazole-based compound and the salicylic acid-based compound in the catalyst, is any ratio in a range of 5: 1-50: 1-50.

Preferably, reaction conditions are as follows: a reaction temperature is any value in a range from 60 to 200°C, a reaction pressure is any value in a range from 0.1MPa to 10.0MPa; and a mass ratio of the catalyst to the alkylene oxide in the raw material is any ratio in a range from 0.001:1 to 1: 1.

Preferably, the molar ratio of the zinc, the imidazole-based compound and the salicylic acid-based compound in the catalyst is any ratio in a range of 5: 2-10: 2-5.

Preferably, the molar ratio of the zinc, the imidazole-based compound and the salicylic acid-based compound in the catalyst, is any ratio selected from 5:1:1, 5:3:5, 5:6:5, 5:10:2, 5:2:3, 5:4:2, 5:7:2, 5:2:2 and 5:50:50, or is any ratio in a range between any two ratios above.

Preferably, the imidazole-based compound is at least one selected from compounds having a chemical formula shown in formula I:

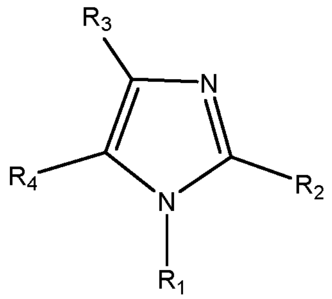

formula I

wherein, in the formula I, $R_1$, $R_2$, $R_3$ and $R_4$ independently from each other are H or $C_1$~$C_{12}$ alkyl.

Preferably, $R_1$, $R_2$, $R_3$ and $R_4$ independently from each other are H or $C_1$~$C_5$ alkyl.

Preferably, $R_1$, $R_2$, $R_3$ and $R_4$ contain zero or one $C_1$~$C_5$ alkyl.

Preferably, the salicylic acid-based compound is at least one selected from compounds having a chemical formula shown in formula II:

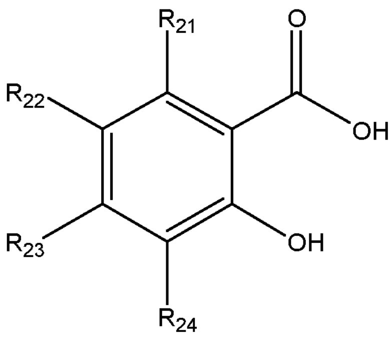

formula II

wherein, $R_{21}$, $R_{22}$, $R_{23}$ and $R_{24}$ independently from each other are H or $C_1$~$C_{12}$ alkyl.

Preferably, the imidazole-based compound is at least one selected from 1-methylimidazole, 2-methylimidazole and butylimidazole.

Preferably, the salicylic acid-based compound is at least one selected from salicylic acid, 3-methyl salicylic acid and 4-methyl salicylic acid.

Preferably, the source of zinc is at least one selected from an oxide of zinc and an inorganic salt of zinc.

Preferably, the source of zinc is at least one selected from zinc chloride, zinc bromide, zinc sulfate, zinc nitrate and zinc oxide.

Preferably, the alkylene oxide is at least one selected from ethylene oxide, propylene oxide, epichlorohydrin oxide and butylene oxide.

Preferably, reaction conditions are as follows: the reaction temperature is any value in a range from 80°C to 160°C, the reaction pressure is any value in a range from 0.5MPa to 8.0MPa, and a mass ratio of the catalyst to the alkylene oxide in the raw material is any ratio in a range from 0.005:1 to 0.5:1.

Preferably, the upper limit of the reaction temperature is 80°C, 100°C, 120°C, 140°C, 160°C, 180°C or 200°C, while the lower limit of the reaction temperature is 60°C, 80°C, 100°C, 120°C, 140°C, 160°C or 180°C.

Preferably, the upper limit of the reaction pressure is 0.2 MPa, 0.5MPa, 0.8MPa, 1.0MPa, 2.0MPa, 3.0MPa, 4.0MPa, 5.0MPa, 6.0MPa, 7.0MPa, 8.0MPa, 9.0 MPa, or 10.0 MPa, while the lower limit is 0.1MPa, 0.2MPa, 0.5MPa, 0.8MPa, 1.0MPa, 2.0MPa, 3.0MPa, 4.0MPa, 5.0MPa, 6.0MPa, 7.0MPa, 8.0MPa, or 9.0MPa.

Preferably, the reacting step comprises: sequentially adding the catalyst and the alkylene oxide in a reactor, feeding carbon dioxide gas to the reactor to perform the reaction under the reaction conditions.

Preferably, a method for preparing the catalyst comprises the following steps:

1) mixing a compound comprising zinc element with a salicylic acid-based compound, adding a solvent therein, and heating to reflux, thereby obtaining a mixture;
2) mixing the mixture with the imidazole-based compound, heating to reflux, and removing the solvent to obtain a solid product which is the catalyst; wherein, a molar ratio of the compound comprising the zinc element, the imidazole-based compound and the salicylic acid-based compound is any ratio in a range of 5: 1-50 : 1-50;

wherein, the mole number of the compound comprising zinc element is counted based on the mole number of the zinc

element.

Preferably, during the preparation of the catalyst, a molar ratio of the compound comprising the zinc element, the imidazole-based compound and the salicylic acid-based compound used in steps 1) and 2) is any ratio selected from 5:1:1, 5:3:5, 5:6:5, 5:10:2, 5:2:3, 5:4:2, 5:7:2, 5:2:2 and 5:50:50, or any ratio in a range between any two above ratios.

Preferably, during the preparation of the catalyst, the compound comprising zinc element used in step 1) is oxide of zinc or inorganic salt of zinc.

Preferably, the solvent is at least one selected from alcohols and ethers.

Preferably, the solvent is at least one selected from methanol, ethanol and diethyl ether.

Preferably, in steps 1) and 2), the reflux time is in a range from 4hours~8 hours.

In the present disclosure, the alkyl in "$C_1$~$C_{12}$ alkyl" refers to an alkyl having 1~12 carbon atoms.

In present disclosure, "alkyl" refers to a group obtained from an alkane by losing one H atom.

The beneficial effects achieved by the present disclosure are as follows: the method adopts a complex catalyst system formed from multidentate ligand and zinc, wherein the multidentate ligand comprises imidazole-based compound and salicylic acid-based compound; a molar ratio of the imidazole-based compound, the salicylic acid-based compound and the zinc in the catalyst is any ratio in a range of 5: 1-50 : 1-50. The catalyst in the present disclosure can catalyze reaction at any temperature in a range from 60°Cto 200°C and any pressure in a range from 0.1 MPa to 10.0 MPa, with a mass ratio of the catalyst to the alkylene oxide being in a range from 0.001:1 to 1:1. The reaction was maintained for 3 hours to obtain 99.5% conversion efficiency of alkylene oxide, 99.2%selectivity of alkylene carbonate, and thus a good technical effect was achieved. The method for preparing the catalyst system is simple, with low cost, and the catalyst exhibits good activity and could be popularized to the industrial production of alkylene carbonate.

## DETAILED DESCRIPTION

The preferred examples of the present disclosure are described below. It should be understood that the preferred examples described herein are only used to illustrate and explain the present disclosure and are not to be construed as limiting the present disclosure.

In these examples, the conversion efficiency of alkylene oxide = (the mole number of alkylene oxide before reaction - the mole number of alkylene oxide after reaction) / the mole number of alkylene oxide before reaction ∗ 100%.

The selectivity of alkylene carbonate = the mole number of alkylene carbonate produced / the total mole of product produced.

### Example 1

0.5mol zinc oxide and 0.5mol salicylic acid were mixed, and 200ml ethanol was then added therein. They were stirred and heated to reflux for 6h, and then 0.3mol imidazole was added therein and mixed. Then the resulting mixture was stirred and heated to reflux for 6h, then cooled and filtered to obtain a catalyst product A.

### Example 2

0.5mol zinc bromide and 0.5mol salicylic acid were mixed, and 200ml ethanol was then added therein. They were stirred and heated to reflux for 6h, and then 0.6mol imidazole was added therein and mixed. Then the resulting mixture was stirred and heated to reflux for 6h, then cooled and filtered to obtain a catalyst product B.

### Example 3

0.5mol zinc chloride and 0.2mol salicylic acid were mixed, and 200ml ethanol was then added therein. They were stirred and heated to reflux for 6h, and then 1mol imidazole was added therein and mixed. Then the resulting mixture was stirred and heated to reflux for 6 hours, then cooled and filtered to obtain a catalyst product C.

### Example 4

0.5mol zinc chloride and 0.3mol salicylic acid were mixed, and 200ml ethanol was then added therein. They were stirred and heated to reflux for 6h. and then 0.2mol 1-methylimidazole was added therein and mixed. Then the resulting mixture was stirred and heated to reflux for 6h, then cooled and filtered to obtain a catalyst product D.

**Example 5**

0.5mol zinc sulfate and 0.2mol salicylic acid were mixed, and 200ml ethanol was then added therein. They were stirred and heated to reflux for 6h, and then 0.4mol 2-methylimidazole was added therein and mixed. Then the resulting mixture was stirred and heated to reflux for 6h, then cooled and filtered to obtain a catalyst product E.

**Example 6**

0.5mol zinc chloride and 0.2mol 3-methyl salicylic acid were mixed, and then 200ml ethanol was then added therein. They were stirred and heated to reflux for 6h, and then 1mol 1-butylimidazole was added and mixed. Then the resulting mixture was stirred and heated to reflux for 6h, then cooled and filtered to obtain a catalyst product F.

**Example 7**

0.5mol zinc nitrate and 0.2mol 4-methyl salicylic acid were mixed, and 200ml ethanol was then added therein. They were stirred and heated to reflux for 6h, and then 0.7mol imidazole was added therein and mixed. Then the resulting mixture was stirred and heated to reflux for 6h, then cooled and filtered to obtain a catalyst product G.

**Example 8**

0.5mol zinc chloride and 0.2mol salicylic acid were mixed, and 200ml ethanol was then added therein. They were stirred and heated to reflux for 6h, and then 0.2mol imidazole was added therein and mixed. Then the resulting mixture was stirred and heated to reflux for 6h, then cooled and filtered to obtain a catalyst product H.

**Comparative Example 1**

0.5mol zinc bromide and 0.2mol salicylic acid were mixed, and then 50ml ethanol was added therein. Then the resulting mixture was stirred and heated to reflux for 6h, then cooled and filtered to obtain a solid intermediate product, thereby obtaining a catalyst product I.

**Comparative Example 2**

0.5mol zinc bromide and 0.2mol imidazole were mixed, and then 50ml ethanol was added therein. Then the resulting mixture was stirred and heated to reflux for 6h, then cooled and filtered to obtain a catalyst product J.

**Comparative Example 3**

0.2mol salicylic acid and 0.2mol imidazole were mixed, and 50ml ethanol was then added therein. Then the resulting mixture was stirred and heated to reflux for 6h, and the ethanol was removed to obtain a catalyst product K.

Comparing three Comparative Examples with Example 8, in the Comparative Example 1, two components were used to obtain catalyst product I, and the catalyst achieved 28.6% conversion efficiency of ethylene oxide ($C_{EO}$) and 24.8% selectivity of an ethylene carbonate ($S_{EC}$), which are shown in table below; in the Comparative Example 2, two components were used to obtain catalyst product J, and the catalyst achieved 47% conversion efficiency of ethylene oxide ($C_{EO}$) and 99% selectivity of an ethylene carbonate ($S_{EC}$), which are shown in table below; in the Comparative Example 3, two components were used to obtain catalyst product K, and the catalyst achieved 4.2% conversion efficiency of ethylene oxide ($C_{EO}$), and 56.3% selectivity of ethylene carbonate ($S_{EC}$). The results show a significant difference from that of Example 8 (the catalyst in Example 8 achieved 99.7% conversion efficiency of ethylene oxide ($C_{EO}$) and 99.6% selectivity of ethylene carbonate ($S_{EC}$).

**Example 9**

0.05g catalyst A and 20mL (0.4mol) ethylene oxide were added into a 100 mL stainless steel autoclave in sequence, then the autoclave was sealed, carbon dioxide with proper pressure was filled therein, the temperature was slowly raised to 140°C by a temperature controller, then the reaction pressure was controlled to be 2.0 MPa, and the reaction was carried out for 2.0 h. After the reaction ended, the reactor was cooled, excessive $CO_2$ was slowly discharged, the reactor was opened, and a small amount of sample was taken for chromatographic analysis. It was tested that the conversion efficiency of ethylene oxide ($C_{EO}$) was 99.5% and the selectivity of ethylene carbonate ($S_{EC}$) was 99.8%.

**Example 10**

0.05g catalyst A and 20 mL (0.4mol) propylene oxide were added into a 100mL stainless steel autoclave in sequence, then the autoclave was sealed, carbon dioxide with proper pressure was filled, the temperature was slowly raised to 120 °C by a temperature controller, then the reaction pressure was controlled to be 2.0 MPa, and the reaction was carried out for 2.0 h. After the reaction ended, the reactor was cooled, excessive $CO_2$ was slowly discharged, the reactor was opened, and a small amount of sample was taken for chromatographic analysis. It was tested that the conversion efficiency of propylene oxide was 99.5% and the selectivity of propylene carbonate was 99.4%.

**Example 11**

0.05g catalyst A and 20 mL (0.4mol) epichlorohydrin were added into a 100mL stainless steel autoclave in sequence, then the autoclave was sealed, carbon dioxide with proper pressure was filled, the temperature was slowly raised to 120°C by a temperature controller, then the reaction pressure was controlled to be 2.0 MPa, and the reaction was carried out for 2.0 h. After the reaction ended, the reactor was cooled, excessive $CO_2$ was slowly discharged, then the reactor was opened, and a small amount of sample was taken for chromatographic analysis. It was tested that the conversion efficiency of epichlorohydrin was 99.3%, and the selectivity of chlorinated propylene carbonate was 99.8%.

**Example 12**

The catalytic reaction of ethylene oxide and carbon dioxide was carried out under the same reaction conditions as those in Example 9, except for the catalyst sample used, the amount thereof and the reaction pressure. The results obtained were shown in Table 1.

Table 1

| Example number | catalyst | The amount of catalyst/g | reaction pressure/MPa | $C_{EO}$/% | $S_{EC}$/% |
|---|---|---|---|---|---|
| 12 | A | 0.05 | 1.0 | 99.5 | 99.8 |
| 13 | A | 0.05 | 3.0 | 99.7 | 99.6 |
| 14 | A | 0.05 | 5 | 99.7 | 99.5 |
| 15 | A | 0.1 | 1.5 | 99.5 | 99.7 |
| 16 | B | 0.05 | 2 | 99.7 | 99.7 |
| 17 | B | 0.1 | 2 | 99.6 | 99.7 |
| 18 | C | 0.05 | 2 | 99.8 | 99.5 |
| 19 | D | 0.05 | 2.0 | 99.7 | 99.6 |
| 20 | E | 0.05 | 2.0 | 99.6 | 99.7 |
| 21 | F | 0.05 | 1.0 | 99.6 | 99.6 |
| 22 | G | 0.05 | 2.0 | 99.8 | 99.7 |
| 23 | H | 0.05 | 2.0 | 99.7 | 99.6 |
| 24 | I | 0.05 | 1.0 | 28.6 | 24.8 |
| 25 | J | 0.05 | 1.0 | 47 | 99.0 |
| 26 | K | 0.05 | 1.0 | 4.2 | 56.3 |

**Examples 27-34**

The catalytic reaction of propylene oxide as raw material and carbon dioxide was carried out under the same reaction conditions as those in Example 10, except that the catalyst sample used was changed. The reaction results obtained were shown in Table 2.

Table 2

| Example number | catalyst | The amount of catalyst/g | reaction pressure/MPa | $C_{PO}$/% | $S_{PC}$/% |
|---|---|---|---|---|---|
| 27 | A | 0.05 | 1.0 | 99.5 | 99.4 |
| 28 | B | 0.05 | 3.0 | 99.3 | 99.6 |
| 29 | C | 0.05 | 2.0 | 99.4 | 99.5 |
| 30 | D | 0.05 | 2.0 | 99.3 | 99.4 |
| 31 | E | 0.05 | 2.0 | 99.5 | 99.5 |
| 32 | F | 0.05 | 1.0 | 99.6 | 99.6 |
| 33 | G | 0.05 | 2.0 | 99.6 | 99.3 |
| 34 | H | 0.05 | 2.0 | 99 7 | 99.4 |

By comparisons, it can be seen that the catalysts in examples of the present disclosure exhibit improved activity or selectivity.

**Example 35**

0.5mol zinc oxide and 0.1mol salicylic acid were mixed, and 200ml ethanol was then added therein. They were stirred and heated to reflux for 6h, and then 0.1mol imidazole was added therein and mixed. Then the resulting mixture were stirred and heated to reflux for 6h, then cooled and filtered to obtain a catalyst product L.

0.05g catalyst L and 20mL (0.4mol) ethylene oxide were added into a 100 mL stainless steel autoclave in sequence, the autoclave was sealed, carbon dioxide with proper pressure was filled, the temperature was slowly raised to 140°C by a temperature controller, then the reaction pressure was controlled to be 2.0 MPa, and the reaction was carried out for 2.0 h. After the reaction ended, the reactor was cooled, excessive $CO_2$ was slowly discharged, the reactor was opened, and a small amount of sample was taken for chromatographic analysis. The conversion efficiency of propylene oxide ($C_{EO}$) was 99.5% and the selectivity of ethylene carbonate ($S_{EC}$) was 99.8%.

**Example 36**

0.5mol zinc oxide and 5mol salicylic acid were mixed, and then 200ml ethanol was added therein. They were stirred and heated to reflux for 6h, and 5mol imidazole was added therein and mixed. Then, the resulting mixture were stirred and heated to reflux for 6h, and then cooled and filtered to obtain a catalyst product M.

0.05g catalyst M and 20 mL (0.4mol) ethylene oxide were added into a 100 mL stainless steel autoclave in sequence, the autoclave was sealed, carbon dioxide with proper pressure was filled, the temperature was slowly raised to 140 °C by a temperature controller, then the reaction pressure was controlled to be 2.0MPa, and the reaction was carried out for 2.0h. After the reaction ended, the reactor was cooled, excessive $CO_2$ was slowly discharged, the reactor was opened, and a small amount of sample was taken for chromatographic analysis. It was tested that the conversion efficiency of propylene oxide ($C_{EO}$) was above 99%, and the selectivity of ethylene carbonate ($S_{EC}$) was above 99%.

In view of the above, although the present disclosure has been described with reference to some preferred examples, it should be understood that the preferred examples described herein are only used to illustrate and are not to be construed as limiting the present disclosure. Various changes and modifications made under the teachings of the present disclosure are equivalent to equivalent examples, without departing from the spirit and scope of the present disclosure.

## Claims

1. A method for preparing alkylene carbonate from alkylene oxide and carbon dioxide, **characterized in that**, the method comprises: contacting a raw material comprising alkylene oxide and carbon dioxide with a catalyst to produce alkylene carbonate;
   wherein, the catalyst is at least one selected from complexes formed from multidentate ligand and zinc;
   the multidentate ligand comprises imidazole-based compound and salicylic acid-based compound; and
   a molar ratio of the zinc, the imidazole-based compound and the salicylic acid-based compound in the catalyst is any ratio in a range of 5: 1-50 : 1-50.

2. The method for preparing alkylene carbonate from alkylene oxide and carbon dioxide according to claim 1, **characterized in that**, reaction conditions are as follows:

   a reaction temperature is any value in a range from 60°C to 200 °C, and a reaction pressure is any value in a range from 0.1MPa to 10.0 MPa;and
   a mass ratio of the catalyst to the alkylene oxide in the raw material is any ratio in a range from 0.001:1 to 1: 1.

3. The method for preparing alkylene carbonate from alkylene oxide and carbon dioxide according to claim 1, **characterized in that**, the molar ratio of zinc, the imidazole-based compound and the salicylic acid-based compound in the catalyst is any ratio in a range of 5: 2-10 : 2-5.

4. The method for preparing alkylene carbonate from alkylene oxide and carbon dioxide according to claim 1, **characterized in that**, the imidazole-based compound is at least one selected from compounds having a chemical formula shown in formula I:

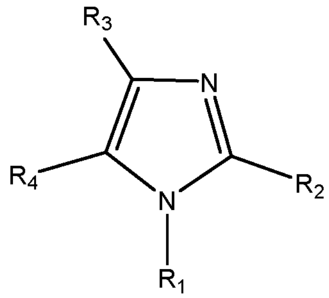
formula I

   wherein, in the formula I, $R_1$, $R_2$, $R_3$ and $R_4$ independently from other are H or $C_1$~$C_{12}$ alkyl.

5. The method for preparing alkylene carbonate from alkylene oxide and carbon dioxide according to claim 4, **characterized in that**, $R_1$, $R_2$, $R_3$ and $R_4$ independently from each other are H or $C_1$~$C_5$ alkyl.

6. The method for preparing alkylene carbonate from alkylene oxide and carbon dioxide according to claim 1, **characterized in that**, the salicylic acid-based compound is at least one selected from compounds having a chemical formula shown in formula II:

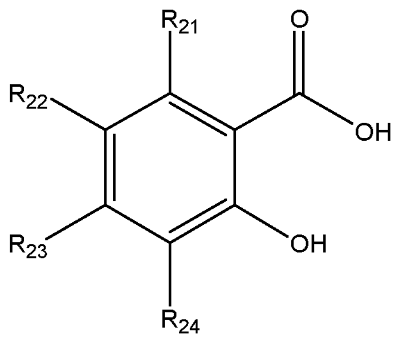
formula II

   wherein, $R_{21}$, $R_{22}$, $R_{23}$ and $R_{24}$ independently from each other are H or $C_1$~$C_{12}$ alkyl .

7. The method for preparing alkylene carbonate from alkylene oxide and carbon dioxide according to claim 1, **characterized in that**, the source of zinc is at least one selected from an oxide of zinc and an inorganic salt of zinc.

8. The method for preparing alkylene carbonate from alkylene oxide and carbon dioxide according to claim 1, **characterized in that**, the source of zinc is at least one selected from zinc chloride, zinc bromide, zinc sulfate, zinc nitrate and zinc oxide.

9. The method for preparing alkylene carbonate from alkylene oxide and carbon dioxide according to claim 1, **characterized in that**, the alkylene oxide is at least one selected from ethylene oxide, propylene oxide, epichlorohydrin

and butylene oxide.

**10.** The method for preparing alkylene carbonate from alkylene oxide and carbon dioxide according to claim 1, **characterized in that**, reaction conditions are as follows:

a reaction temperature is any value in a range from 80°C to 160 °C, and a reaction pressure is any value in a range from 0.5 MPa to 8.0 MPa; and
a mass ratio of the catalyst to the alkylene oxide in the raw material is any ratio in a range from 0.005 :1 to 0.5: 1.

**11.** The method for preparing alkylene carbonate from alkylene oxide and carbon dioxide according to claim 1, **characterized in that**, the reacting step comprises: sequentially adding the catalyst and the alkylene oxide in a reactor, and feeding carbon dioxide gas to the reactor to perform the reaction under the reaction conditions.

**12.** The method for preparing alkylene carbonate from alkylene oxide and carbon dioxide according to claim 1, **characterized in that**, a method for preparing the catalyst comprises following steps:

1) mixing a compound comprising zinc element with a salicylic acid-based compound, adding a solvent therein, and heating to reflux to obtain a mixture;
2) mixing the mixture with an imidazole-based compound, heating to reflux, removing the solvent to obtain a solid product which is the catalyst; wherein, a molar ratio of the compound comprising the zinc element, the imidazole-based compound and the salicylic acid-based compound is any ratio in a range of 5: 1-50: 1-50;

wherein, the mole number of the compound comprising zinc element is counted based on the mole number of the zinc element.

**13.** The preparation method according to claim 12, **characterized in that**, the solvent is at least one selected from alcohols and ethers.

**14.** The preparation method according to claim 12, **characterized in that**, the solvent is at least one selected from methanol, ethanol and diethyl.

**15.** The preparation method according to claim 12, **characterized in that**, in the heating to reflux of steps 1) and 2), reflux time is in a range from 4 hours to 8 hours.

**INTERNATIONAL SEARCH REPORT**

International application No.
**PCT/CN2020/075766**

**A. CLASSIFICATION OF SUBJECT MATTER**

C07D 317/38(2006.01)i; B01J 31/22(2006.01)i

According to International Patent Classification (IPC) or to both national classification and IPC

**B. FIELDS SEARCHED**

Minimum documentation searched (classification system followed by classification symbols)

C07D; B01J

Documentation searched other than minimum documentation to the extent that such documents are included in the fields searched

Electronic data base consulted during the international search (name of data base and, where practicable, search terms used)

VEN; CNABS; CNKI; STN (CAPLUS): 碳酸烷基酯, 碳酸酯, 环氧烷烃, 环氧, 二氧化碳, 催化剂, 锌, 咪唑, 水杨酸, catalyst, ethylene , carbonate, zinc, Zn

**C. DOCUMENTS CONSIDERED TO BE RELEVANT**

| Category* | Citation of document, with indication, where appropriate, of the relevant passages | Relevant to claim No. |
|---|---|---|
| Y | SHI, Yanli et al. "Homogenous Dual-Ligand Zinc Complex Catalysts for Chemical Fixation of CO2 to Propylene Carbonate" *Catal. Lett.,* Vol. 145, 16 July 2015 (2015-07-16), pp. 1673-1682, formula 1, table 1, and section 2, experimental part | 1-15 |
| Y | 林吟卿 等 (LIU, Yinqing et al.). "锌(II)与2-甲基咪唑和水杨酸络合物的结构分析 (Structural Analysis of Complex Formed by Zn(II) with 2-Methylimidazole and O-Hydroxybenzoic Acid)" *分析测试通报 (Journal of Instrumental Analysis),* Vol. 8, No. 2, 31 December 1989 (1989-12-31), p. 51, the abstract | 1-15 |
| A | CN 104492488 A (NANJING TECH UNIVERSITY; NANJING KAINAISI CHEMICAL SCIENCE & TECHNOLOGY CO., LTD.; SHANDONG DEPU CHEMICAL INDUSTRY SCIENCE AND TECHNOLOGY CO., LTD.) 08 April 2015 (2015-04-08) entire document | 1-15 |
| A | WO 2014157524 A1 (TAKASAGO PERFUMERY CO LTD) 02 October 2014 (2014-10-02) entire document | 1-15 |
| A | CN 108772102 A (LANZHOU UNIVERSITY) 09 November 2018 (2018-11-09) entire document | 1-15 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 May 2020** | **15 May 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2020/075766**

| Patent document cited in search report | Publication date (day/month/year) | Patent family member(s) | Publication date (day/month/year) |
|---|---|---|---|
| CN 104492488 A | 08 April 2015 | CN 104492488 B | 26 April 2017 |
| WO 2014157524 A1 | 02 October 2014 | DE 112014001645 T5 | 21 January 2016 |
| | | US 2016002268 A1 | 07 January 2016 |
| | | JP WO2014157524 A1 | 16 February 2017 |
| | | JP 6484876 B2 | 20 March 2019 |
| | | US 9611279 B2 | 04 April 2017 |
| CN 108772102 A | 09 November 2018 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2907771 A
- US 7488835 B
- CN 1995032